# EUROPEAN PATENT SPECIFICATION

(11) **EP 0 376 904 B1**
(45) Date of publication and mention of the grant of the patent: **25.05.1994**
(21) Application number: 89830567.7
(22) Date of filing: 22.12.1989
(51) Int. Cl.: A61N 1/36, H01R 4/56

(54) **A connector device, particularly for connecting an elecrical catheter to a heart pacemaker**
Verbindungsvorrichtung, besonders zur Verbindung eines elektrischen Katheteres mit einem Herzschrittmacher
Connecteur, en particulier pour raccorder un cathéter électrique à un stimulateur cardiaque

(30) Priority: 29.12.1988 IT 5365088 U
(43) Date of publication of application: 04.07.1990
(73) Proprietor: SORIN BIOMEDICA CARDIO S.p.A., I-10125 Torino (IT)
(72) Inventor: Rolando, Giovanni, I-10034 Chivasso (Torino) (IT)
(74) Representative: Rambelli, Paolo

(56) References cited:
- DE-B- 1 216 399
- DE-U- 8 019 782
- FR-A- 2 504 383

## Description

The present invention relates to a system comprising a pacemaker and a connector device for connecting a pacing lead comprising at least one wire wound into helical turns to the pacemaker.

DE-U-80 19 782 describes an electrical connector device for high voltage comprising a conical screw member and a cylindrical internally threaded socket member.

The particular object of the invention is to provide a connector device which enables a permanent, irreversible mechanical and electrical connection to be made between an electrical catheter and a heart pacemaker by a simple operation so that a worn-out heart pacemaker can be replaced quickly and easily by a doctor.

A further object is to provide a connector which can act as an adaptor so as to enable an electrical catheter and its implanted electrode to be connected to a new heart pacemaker with a different input attachment configuration from that of the heart pacemaker used previously.

A further object is to provide a connector which enables a heart pacemaker to be connected to an electrical catheter by a connection with very little radial bulk.

These objects are achieved by means of a system comprising at least a pacemaker having a connector device and a helically wound pacing lead, characterised in that said connector is constituted by a body of conductive material which has a threaded end part which tapers towards its proximal end and which is screwed into the turns of the end portion of the pacing lead, which expands resiliently.

By virtue of these characteristics, the connection can be achieved by a simple screwing operation and, the use of the resilient deformation of the wire, ensures very good mechanical and electrical connection.

Further characteristics and advantages will become clear from the detailed description which follows with reference to the appended drawings, in which:
Figure 1 is a schematic view of a heart pacemaker with the catheter connecting it to a pacemaker electrode;
Figure 2 is a front elevational view of a connector according to the invention for connecting an electrical catheter to a heart pacemaker of the type of Figure 1, and
Figure 3 is an exploded view of a detail taken on the arrow III of Figure 1, on an enlarged scale.

With reference to the drawings, a connector device according to the invention, generally indicated 2, is constituted by an elongate body of conductive material with an end part 6 constituting a shank for connection to a heart pacemaker ES and an end part 4 for connection to an electrical catheter EC.

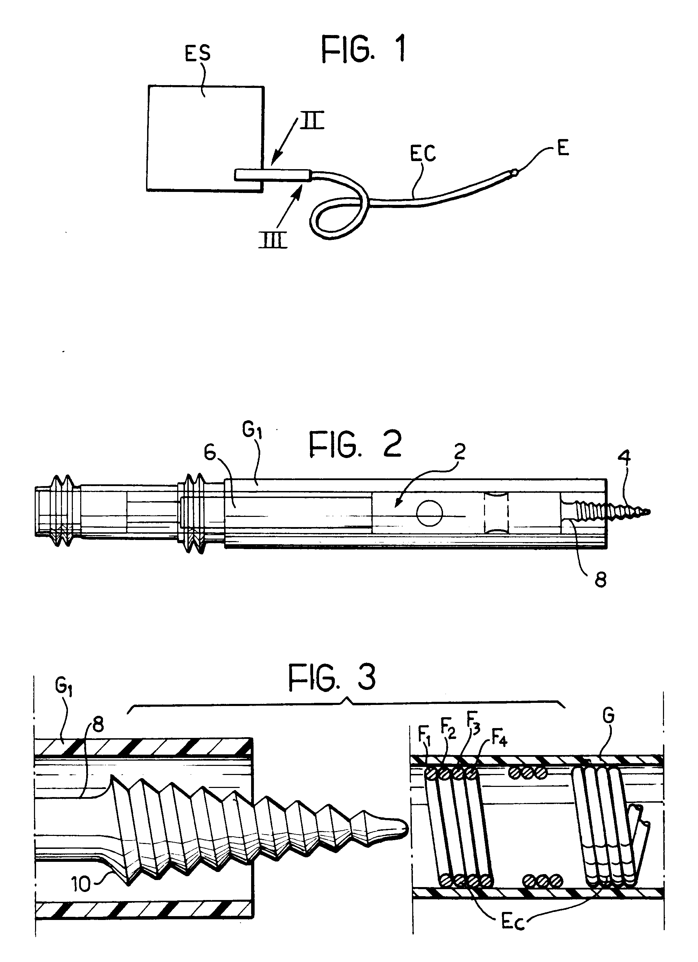

According to the invention, the end part 4 is threaded and conically-tapered towards the end which, in the condition of use, is proximal to the body in which a pacemaker electrode E is implanted. The end part 4 is intended to be screwed into the turns of the electrical catheter EC. The electrical catheter is generally constituted by one or more conductive wires indicated F₁, F₂, F₃, F₄, wound in cylindrical helices. In order to improve the electrical contact and the mechanical connection, the end part 4 preferably has a different number of thread starts from the number of wires constituting the electrical catheter. A two-start thread is preferred, particularly for an electrical catheter constituted by four wires such as that shown in Figure 3. A right-hand thread is used for electrical catheters with right-hand turns and vice versa.

In the preferred embodiment, the connector has a neck portion, indicated 8 adjacent the base surface of the threaded conical end part, which defines a shoulder 10 for stopping the turns of the conductor or electrical pacing lead EC. The end part 4 is typically 4-5 mm long with a taper of between 3 and 12°, preferably 5-6°. As it can be seen, the conical shape enables an effective connection to be made use with any electrical catheter whose external diameter falls within a certain range. The same connector can therefore be used for connection to electrical catheters of different dimensions.

The replacement of a worn-out heart pacemaker can be carried out particularly easily by a cardiologist. For this purpose, after he has cut off the electrical catheter EC adjacent the heart pacemaker ES leaving the stimulator electrode E in place, the cardiologist connects a new heart pacemaker to the electrical catheter by means of the connector device according to the invention. This operation is carried out by the insertion of the threaded part of the connector into the turns of the electrical catheter which is in turn fitted into a protective sheath G of plastics material, the threaded part being rotated, generally in a clockwise sense, so that the turns screw on to, and rise over, the threads with progressive resilient radial deformation. This means that the turns become jammed in the recesses of the threads so that they cannot be removed by a simple rotary movement in the opposite sense. When the turns of the electrical catheter pass over the last thread they close resiliently against the shoulder 10, further increasing the irreversibility of the connection. The radial tightening of the coils on to the conical end part also ensures good electrical contact. The connector 2, which is also fitted in a sheath G1, is then connected to a new heart pacemaker by means of the terminal 6. The sheath G of the electrical catheter, which is inserted in the sheath G1 surrounding the end portion 4, is fixed to the sheath G1 by means of silicone glue.

## Claims

1. A system comprising in combination a pacemaker (ES) having a connector device for connecting a pacing lead, and a helically wound pacing lead (EC), characterised in that said connector is constituted by a body (2) of conductive material with a threaded end part (4) which tapers towards its proximal end and is screwed into the turns of the end portion of the pacing lead, which expand resiliently.

2. A system according to Claim 1, characterised in that the end part of said connector is a circular-sectioned cone.

3. A system according to Claim 1, characterised in that, at its end opposite the conical end, the body of said connector has an attachment shank (6) for connection to the pacemaker.

4. A system according to any one of Claims 1 to 3, characterised in that the conical end of said connector has a screw thread with a different number of thread starts from the number of wires constituting the pacing lead which is connected thereto.

5. A system according to any one of Claims 1 to 4, characterised in that the screw thread of said connector has two starts.

6. A system according to any one of Claims 1 to 5, characterised in that the conical end of said connector has a taper of between 3° and 12°.

7. A system according to Claim 6, characterised in that the conical end has a taper of between 5° and 6°.

8. A system according to any one of Claims 1 to 7, characterised in that, adjacent the base surface of the tapered end, said connector has a neck portion (8) defining a shoulder (10) for stopping the turns of the pacing lead.

9. Use of a connector device as described in Claim 1, for connecting a heart pacemaker to a helically wound pacing lead.

## Patentansprüche

1. System, das in einer Kombination einen Schrittmacher (ES), der eine Steckereinrichtung für den Anschluß eines Schrittmacherkabels besitzt, sowie ein schraubenförmig gewundenes Schrittmacherkabel (EC) enthält, dadurch gekennzeichnet, daß der Stecker von einem Körper (2) aus einem leitenden Material gebildet wird, der einen Gewindeendteil (4) besitzt, der sich zu seinem körpernahen Ende verjüngt und in die Windungen des Endteils des Schrittmacherkabels geschraubt wird, der sich elastisch erweitert.

2. System gemäß Anspruch 1, dadurch gekennzeichnet, daß der Endteil des Steckers ein Kreiskegel ist.

3. System gemäß Anspruch 1, dadurch gekennzeichnet, daß der Körper des Steckers an seinem dem Kegelende gegenüberliegenden Ende einen Befestigungsschaft (6) für eine Verbindung mit dem Schrittmacher besitzt.

4. System gemäß irgendeinem der Ansprüche 1 bis 3, dadurch gekennzeichnet, daß das Kegelende des Steckers ein Schraubengewinde besitzt, das eine andere Anzahl von Gewindegängen besitzt, als Drähte vorhanden sind, die das Schrittmacherkabel bilden, das damit verbunden ist.

5. System gemäß irgendeinem der Ansprüche 1 bis 4, dadurch gekennzeichnet, daß das Schraubengewinde des Steckers zwei Gewindegänge besitzt.

6. System gemäß irgendeinem der Ansprüche 1 bis 5, dadurch gekennzeichnet, daß das Kegelende des Steckers eine Verjüngung zwischen 3° und 12° besitzt.

7. System gemäß Anspruch 6, dadurch gekennzeichnet, daß das Kegelende eine Verjüngung zwischen 5° und 6° besitzt.

8. System gemäß irgendeinem der Ansprüche 1 bis 7, dadurch gekennzeichnet, daß der Stecker neben der Grundfläche des Kegelendes einen Halsteil (8) besitzt, der eine Schulter (10) bildet, um die Windungen des Schrittmacherkabels aufzuhalten.

9. Verwendung einer Steckereinrichtung, wie sie im Anspruch 1 beschrieben wurde, um einen Herzschrittmacher mit einem schraubenförmig geschlungenen Schrittmacherkabel zu verbinden.

## Revendications

1. Système comprenant, en combinaison, un stimulateur (Es) présentant un connecteur pour raccorder un conducteur d'entraînement électrosystolique et un conducteur d'entraînement électrosystolique (EC) enroulés hélicoïdalement caractérisé en ce que ledit connecteur est constitué d'un corps (2) en matériau conducteur, dont une partie d'extrémité (4) filetée va en s'effilant vers son extrémité proximale et est vissée dans les tours de la partie d'extrémité du conducteur d'entraînement électrosystolique, qui s'étend élastiquement.

2. Système selon la revendication 1, caractérisé en ce que la partie d'extrémité dudit connecteur est un cône à section transversale circulaire.

3. Système selon la revendication 1, caractérisé en ce qu'à son extrémité opposée à l'extrémité conique le corps dudit connecteur présente un fût de fixation (6) destiné à être relié au stimulateur cardiaque.

4. Système selon l'une quelconque des revendications 1 à 3 caractérisé en ce que l'extrémité conique dudit connecteur présente un filetage pourvu d'un nombre de débuts de filetage différent du nombre de fils constituant le conducteur d'entraînement électrosystolique qui est relié à ce dernier.

5. Système selon l'une quelconque des revendications 1 à 4, caractérisé en ce que le filetage dudit connecteur présente deux débuts.

6. Système selon l'une quelconque des revendications 1 à 5, caractérisé en ce que l'extrémité conique dudit connecteur présente un effilement compris entre 3° et 12°.

7. Système selon la revendication 6, caractérisé en ce que l'extrémité conique présente un effilement compris entre 5° et 6°.

8. Système selon l'une quelconque des revendications 1 à 7, caractérisé en ce que, de manière adjacente à la surface de base de l'extrémité effilée, ledit connecteur présente une partie de col (8) définissant un épaulement (10) destiné à stopper la rotation du conducteur d'entraînement électrosystolique.

9. Utilisation d'un connecteur selon la revendication 1, servant à raccorder un stimulateur cardiaque à un conducteur d'entraînement électrosystolique enroulé hélicoïdalement.
